# EUROPEAN PATENT SPECIFICATION

(11) **EP 2 939 618 B1**
(45) Date of publication and mention of the grant of the patent: **31.01.2018**
(21) Application number: 13868685.2
(22) Date of filing: 25.07.2013
(51) Int. Cl.: A61B 17/3201, A61B 17/28, A61B 17/32, A61B 17/00, A61B 17/30, A61B 17/285, A61B 17/295

(54) **SURGICAL INSTRUMENT**
CHIRURGISCHES INSTRUMENT
INSTRUMENT CHIRURGICAL

(30) Priority: 27.12.2012 JP 2012284154
(43) Date of publication of application: 04.11.2015
(73) Proprietor: Charmant Inc., Sabae City Fukui 916-8555 (JP)
(72) Inventor: OHKI, Takao, Tokyo 105-0002 (JP)
(74) Representative: Pitchford, James Edward
(86) International application number: PCT/JP2013/004528
(87) International publication number: WO 2014/103096

(56) References cited:
- EP-A1- 0 965 309
- WO-A1-2006/041317
- FR-A- 1 183 358
- JP-A- H11 137 563
- JP-A- 2000 210 296
- JP-A- 2000 210 296
- US-A- 2 315 326
- US-A- 5 342 381
- US-A- 5 383 471
- US-A- 5 439 471
- US-A1- 2003 065 358

## Description

### Technical Field

The present invention relates to a surgical instrument, particularly to a surgical instrument having a plurality of functions.

### Background Art

In a surgery, a series of surgical operations is performed as follows. After the epidermis is lightly incised, a site where important living tissues such as a blood vessel or a nerve exist is treated such that living tissues are separated from each other using forceps or the like, rarely using a scalpel, to prevent impairing the living tissues. When a surgical site has been reached, the surgical site is incised or resected as required by using a scalpel and scissors. Thereafter, a needle is grasped with forceps to be used for suture and ligation and then the suture is cut off. As described above, basic operations in a surgery include: separation of living tissues; grasping of the living tissues, needles, and suture or the like; and cutting of the living tissues and suture or the like. A surgery is a repetition of a series of these surgical operations, and a dedicated surgical instrument is used corresponding to each surgical operation. Accordingly, surgical instruments are frequently exchanged in a surgery, and a surgical instrument held by a practitioner is frequently exchanged with another by the practitioner.

Patent Literature 1 describes as one of the surgical instruments used in a surgery as "forceps having a configuration with a detachable soft cover member at a clamping section thereof, the soft cover member being sterilized beforehand, to be provided as disposable forceps that can be manufactured at low cost" (paragraph 0005 in Patent Literature 1) as a technical problem. This Literature also describes "...forceps configured to attach a disposable and detachable cylindrical soft cover member to each clamping section" (Claim 1 in Patent Literature 1).

Also Patent Literature 2 describes, as a technical problem, "to provide a mucosa dissection assistance device which is inserted beside an endoscope to avoid making a perforation or a stab wound on a surface of a skin for performing counter-traction of a mucosa to be resected and which enables smooth release of the mucosa after removal of the mucosa without having the mucosa caught in a section for grasping the mucosa." (paragraph 0005 in Patent Literature 2). The Literature also describes "... to provide a mucosa dissection assistance device including: a mucosa grasping section, a flexible pipe having the mucosa grasping section on one end; an elastic member passing from an end of the pipe through the inside of the tubular member, passing again from the end of the pipe through the inside of the pipe, which is longitudinally extensible; and an operating line passing inside the pipe, one end being connected to one and the other end of the elastic member, and that operates to make an annular diameter of the mucosa grasping section variable via the elastic member" (Claim 1 in Patent Literature 2).

Although various surgical instruments have been developed other than instruments described in Patent Literatures 1 and 2, it is desirable to further develop a surgical instrument for decreasing a burden on a patient and health-care professionals and ensuring convenience and safety.

### Citation List

### Patent Literature

Patent Literature 1: JP 08-224251 A
Patent Literature 2: JP 2007-307000 A

Further background art is provided in US 2 315 326 A, US 5 439 471 A, JP 2000 210296 A, US 5 383 471 A, FR 1 183 358 A and US 2003/065358 A1.

US 2 315 326 A discloses a surgical instrument which can be used for suturing cuts or wounds, and which is adapted to guide a needle and thereafter sever the thread between stitches.

US 5 439 471 A discloses a combined surgical needle holder and scissors for use in suturing procedures. The instrument includes a pair of arms which are pivotally connected together at a location intermediate their ends. A flat clamping jaw is associated with each arm and is spaced from a first end of the arm. As the arms are pivoted, the jaws are brought into clamping relation to clamp a surgical needle. A scissors blade is formed on each arm and is located between the clamping jaw and the distal end of the arm. The scissor blades are constructed to be moved into overlapping cutting relation when the distal ends of the arms are moved toward each other.

JP 2000 210296 A discloses a medical treatment tool. A holding part to hold tissue is disposed on the tip end parts of each of the opening/closing elements of the forceps main body. A wide contact plate formed of a metallic material with high heat conductivity is placed on the inner face side of the holding part, with a heat generating element inside. The heat generating element is fixed on the inner face of each contact plate so that heat of the heat generating element is transferred to the holding part via the contact plate when electricity is applied to the heat generating element to heat and coagulate the tissue held in between the holding parts. A half side scissors blade, for instance made of a stainless steel material, is integrally formed with a back plate member to the rear side of each holding part to form an incising part to incise tissue.

US 5 383 471 A discloses a surgical instrument usable as a biopsy tool having two or more complementary mating jaws for securing and cutting the selected tissue, pivoted and operable remote from the operator handle. The surgical instrument includes elements to prevent the relative dislocation of the cutting elements when the instrument is rotated on an axis perpendicular to the pivot point of the jaws. Further features include an asymmetric continuous cutting surface about the major portion of the perimeter of the jaws, and a protruding tip of each of the jaw cutting surfaces, forming an incisor-like cutting region to enable the selected tissue to be secured within the region of the jaw and cut free from the surrounding tissue. When fully engaged, the surgical instrument encloses the sample to be removed.

FR 1 183 358 A discloses a surgical device comprising scissors and a clamp.

US 2003/065358 A1 discloses an apparatus for minimally invasive surgery. The apparatus comprises a tool which includes a multi-functioning end effector for insertion into a patient, a user control adapted for use by the surgeon external of the patient, and an intermediate section between the end effector and the user control to translate control instructions from the user control through an actuating mechanism to operate the end effector in one of at least two different functioning states.

### Summary of Invention

### Technical Problem

As described above, a surgery includes a series of repetitive surgical operations of separation, grasping, and cutting, and a surgical instrument is frequently exchanged with a dedicated instrument corresponding to each surgical operation. Such frequent exchanges of surgical instruments become a factor of a prolonged surgery, which increases a burden on a patient and practitioners. Furthermore, using multiple surgical instruments of different types complicates management of the surgical instruments.

The present invention is intended to solve such a problem. It is an object of the present invention to decrease the types of surgical instruments in a surgery and provide a surgical instrument capable of decreasing a burden on a patient and health-care professionals involving in the surgery.

### Solution to Problem

The present invention is a surgical instrument as defined in Claim 1 of the appended claims. Preferred embodiments are defined in the dependent claims.

Solutions to the above problem includes:
(1) a surgical instrument having a pair of working sections openably/closably coupled to each other on a pivot shaft, the pair of working sections including:
   a cutting section configured to cut an object by sliding a pair of cutting edges against each other;
   a pair of grasping surfaces configured to grasp the object by closing the pair of working sections; and
   a pair of separating sections configured to be able to separate living tissues when opening the pair of working sections.

Preferable aspects of (1) above includes:
(2) the surgical instrument according to (1), including a step section without a blade between the grasping surface and the cutting edge;
(3) the surgical instrument according to the above (1) or (2), wherein, on a pair of rod-like members configured to cross each other around the pivot shaft as an intersection, the pair of working sections is provided on a tip side with respect to the pivot shaft, a pair of operating sections is provided on a rear end side with respect to the pivot shaft, and the pair of working sections is configured to open and close corresponding to opening and closing of the pair of operating sections; and
(3) the surgical instrument according to any one of the above (1) to (3), wherein the grasping section is substantially elliptical, an axial length in a longitudinal direction of the rod-like member and an axial length crossing the axial length in an orthogonal direction each are from 1 to 5 mm, a sum of thicknesses of a pair of tip sections in a closed state of the pair of working sections is from 1 to 5 mm, and a length of the cutting section is from 5 to 50 mm.

### Advantageous Effects of Invention

A surgical instrument according to the present invention has three functions enabling: cutting an object such as a living tissue and suture by the cutting section; grasping an object such as a living tissue and suture by the grasping surface; and separating living tissues from each other by the separating section.

Compared with a conventional surgery in which a dedicated surgical instrument has been used for each surgical operation of separating, grasping, and cutting, the above-described surgical instrument of the present invention alone can achieve the above-described three surgical operations, making it possible to decrease the number of types of surgical instruments used in a surgery. As a result, management of surgical instruments can be streamlined, burden on health-care professionals being decreased, making it possible to reduce a total cost.

Also, conventionally, a surgical instrument has been exchanged with a dedicated surgical instrument corresponding to each surgical operation, and a surgical instrument held by a practitioner has been frequently exchanged with another by the practitioner. A surgical instrument of the present invention can perform the three surgical operations alone, making it possible to decrease the number of times of exchanging surgical instruments by the practitioner, thereby reducing the time taken for a surgery. Reducing the time taken for a surgery not only decreases the burden on a patient by suppressing the amount of bleeding or the like, but also decreases a burden on all health-care professionals involving in the surgery.

### Brief Description of Drawings

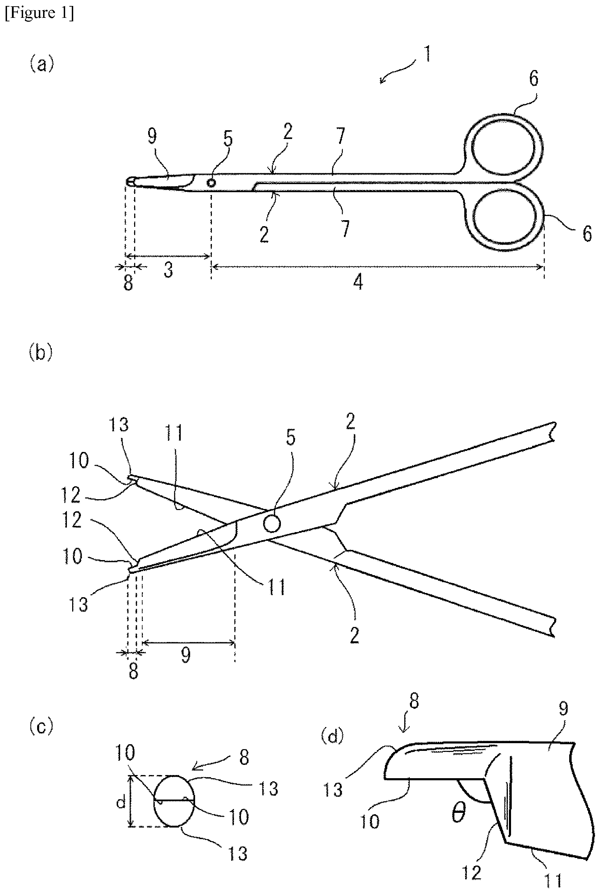

Figs. 1(a) to 1(d) are schematic diagrams illustrating a surgical instrument according to an embodiment of the present invention. Fig. 1(a) is a top-view schematic diagram illustrating a closed state of a surgical instrument according to an embodiment of the present invention. Fig. 1(b) is a top-view schematic diagram illustrating main portions of the surgical instrument illustrated in Fig. 1(a), including an enlarged view of a working section in an open state. Fig. 1(c) is a side-view schematic diagram, illustrating the surgical instrument illustrated in Fig. 1(a) as seen from the tip side. Fig. 1(d) is a side-view schematic diagram illustrating main portions, including an enlarged view of a tip section, of the surgical instrument illustrated in Fig. 1(a).
Fig. 2 is a schematic diagram illustrating a surgical instrument according to another embodiment of the present disclosure.
Fig. 3 is a schematic diagram illustrating an embodiment of a medical robot system having the surgical instrument of the present disclosure mounted as a manipulator.
Fig. 4 is a schematic diagram illustrating an enlarged view of main portions of a manipulator.
Fig. 5 is a schematic diagram illustrating a surgical instrument according to an embodiment of the present disclosure
when the surgical instrument is used as an endoscopic surgical instrument.

Figs. 6(a) and 6(b) are schematic diagrams illustrating enlarged views of main portions of the endoscopic surgical instrument illustrated in Fig. 5. Fig. 6(a) is a schematic diagram illustrating main portions of the endoscopic surgical instrument illustrated in Fig. 5, in an open state. Fig. 6(b) is a schematic diagram illustrating main portions of the endoscopic surgical instrument in Fig. 5 in a closed state.

### Description of Embodiments

Hereinafter, a surgical instrument of the present disclosure will be described with reference to the drawings. Fig. 1(a) is a top-view schematic diagram illustrating a closed state of a surgical instrument according to an embodiment of the present invention. Fig. 1(b) is a top-view schematic diagram illustrating main portions of the surgical instrument illustrated in Fig. 1(a), including an enlarged view of a working section in an open state. Fig. 1(c) is a side-view schematic diagram, illustrating the surgical instrument illustrated in Fig. 1(a) as seen from the tip side.

As illustrated in Fig. 1, a surgical instrument 1 of the present embodiment includes a pair of rod-like members 2 crossing each other with respect to a pivot shaft 5 as an intersection. On the pair of rod-like members 2, working sections 3, 3 are provided at a tip side with respect to the pivot shaft 5, and operating sections 4, 4 operated by inserting a finger are provided at a rear end side with respect to the pivot shaft 5. The pair of working sections 3, 3 is coupled to each other openably/closably on the pivot shaft 5. The pair of working sections 3, 3 includes: cutting sections 9, 9 configured to cut an object by sliding a pair of cutting edges 11 against each other; a pair of grasping surfaces 10, 10 configured to grasp the object by closing the pair of working sections 3, 3; and a pair of separating sections 13 configured to be able to separate living tissues when opening the pair of working sections 3, 3. The pair of working sections 3, 3 opens and closes according to the opening and closing of the pair of operating sections 4, 4. The surgical instrument 1 has a mechanism that is similar to ordinary scissors in that a rotation shaft 5 works as a fulcrum, the operating sections 4, 4 working as points of force, and the working sections 3, 3 working as points of action.

The rod-like member 2 may be formed of a material that is generally used as a material of medial forceps and scissors, the material including, for example, titanium, titanium alloy such as Ti6Al4, stainless steel, and cobalt chromium steel. Furthermore, the surgical instrument 1 preferably has all outer surface corners thereof rounded to avoid impairing a living tissue.

The operating sections 4, 4 are grasped by a health-care professional, and the working sections 3, 3 are opened and closed by opening and closing of the pair of operating sections 4, 4. The operating sections 4, 4 include ring-shaped ring sections 6, 6 for finger insertion and handle sections 7, 7 that extend from the ring sections 6, 6.

The working sections 3, 3 have three functions including: separating living tissues from each other in a surgery; grasping a surgical instrument such as a needle and suture, and a living tissue; and cutting a living tissue and suture or the like. The working sections 3, 3 include, in the order from the tip side, tip sections 8, 8 having the separating sections 13, 13 and the grasping surfaces 10, 10, a step section 12, and the cutting sections 9, 9.

The separating sections 13, 13 are configured to be able to separate living tissues when opening the pair of working sections 3, 3. The separating sections 13, 13, as illustrated in Figs. 1(a) and 1(c), are outer surfaces on tip sides when the pair of working sections 3, 3 is in a closed state. The outlines of the separating sections 13, 13 have rounded shapes. The surgical instrument 1 can separate living tissues from each other by changing the state of the pair of working sections 3, 3 from closed to open. Since the separating sections 13, 13, which are the outer surfaces of the tip sections 8, 8 and come in contact with the living tissues, have rounded shapes, the surgical instrument 1 can separate the living tissues from each other without impairing crucial living tissues. As illustrated in Fig. 1(c), the separating sections 13, 13 have substantially elliptical outlines when the pair of working sections 3, 3 is closed, viewed from the tip side of the surgical instrument 1. The outline may be a vertically-long ellipse or a horizontally-long ellipse. A total thickness d of a pair of tip sections 3, 3 is preferably from 1 to 5 mm in view of operability in separation. Also, although the outline of the separating sections 13, 13 of the present embodiment is substantially elliptical, the shape may be a circle, oval, a rounded square, or a rounded rectangle, or the like. The separating sections 13, 13 may have flat outer surfaces at opposite sides of the grasping surfaces 10, 10 where the pair of tip sections 8, 8 face each other. Furthermore, as illustrated in Fig. 1(d), the separating sections 13, 13 have rounded outlines with no corners when viewed from the side of the surgical instrument 1. The outlines of the separating sections 13, 13, for example, make an arc having a curvature radius ranging from two fifths to three fifths of the total thickness d starting from the tip to the rear end, being connected smoothly to the cutting sections 9, 9 having linear outlines.

The grasping surfaces 10, 10 are configured to be able to grasp an object by closing the pair of working sections 3, 3. The grasping surfaces 10, 10, as illustrated in Fig. 1(a) and (c), are configured such that the surfaces facing each other fittingly overlap when the pair of working sections 3, 3 is in a closed state. At this time, the grasping surfaces 10, 10 are orthogonal to the opening/closing direction of the working sections 3, 3. The surgical instrument 1 can grasp or clamp an object such as a surgical instrument including a needle and suture and such as a living tissue after having the object disposed between the pair of grasping surfaces 10, 10, and then having the pair of working sections 3, 3 closed. The grasping surfaces 10, 10 are only required to have areas capable of grasping the object. For example, if an outline of the grasping surface 10 viewed from above is substantially elliptical, an axial length in a longitudinal direction of the working section 3 and an axial length in a direction orthogonal to the above-mentioned axial length each are preferably from 1 to 5 mm. Note that the outline of the grasping surface 10 viewed from above is not limited to substantially elliptical; the shape may be a circle, oval, a rounded square, or a rounded rectangle, or the like. Furthermore, the surfaces of the grasping surfaces 10, 10 may have suitable degree of irregularities for easily grasping the object.

The cutting sections 9, 9 are configured to be able to cut the object by sliding a pair of cutting edges against each other. The cutting sections 9, 9 are provided between the tip sections 8, 8 and the rotation shaft 5, and includes the cutting edges 11, 11 on the sides facing each other on the pair of cutting sections 9, 9. The surgical instrument 1 can cut an object such as a living tissue or suture, after having the object disposed between the pair of cutting sections 9, 9, by closing the pair of working sections 3, 3 to slide the pair of cutting edges 11, 11 against each other. The cutting edges 11, 11 preferably have the lengths from 5 to 50 mm according to the site for which the surgical instrument 1 is used.

The step sections 12, 12 are provided between the grasping surfaces 10, 10 and the cutting edges 11, 11. The step sections 12, 12 are provided as a result of a difference in thickness, that is, the thickness compared in the opening/closing direction on the pair of working sections 3, 3 is larger at the cutting sections 9, 9 than at the tip sections 8, 8. The step sections 12, 12 start from the end portion opposite to the tip side of the grasping surfaces 10, 10, extending toward the cutting sections 9, 9 with inclination, to be connected to the cutting edges 11, 11. The step sections 12, 12 have rounded shapes, i.e. R shapes, and have no blades. The step sections 12, 12 are not always necessary in the surgical instrument of the present invention. Having the step section 12, however, is beneficial in that the step sections 12, 12 become walls when an object is grasped by the grasping surfaces 10, 10, thereby preventing the object to be grasped by the grasping surfaces 10, 10 from being erroneously disposed and cut between the cutting edges 11, 11. Also, the step sections 12, 12 have no blades, so that the object may not be cut at the step sections 12, 12. Note that the step sections 12, 12 are only required to be boundaries that functionally divide the grasping surfaces 10, 10 from the cutting edges 11, 11. Heights of the step sections 12, 12 in a direction orthogonal to the grasping surfaces 10, 10 are preferably 0.1 mm or more, and preferably 10 mm or less considering the relationship between the thickness of the tip sections 8, 8 and the thickness of the cutting sections 9, 9. Furthermore, regarding the step sections 12, 12, when defining an angle formed between the grasping surface 10 and the step section 12 as θ as illustrated in Fig. 1(d), the angle θ is preferably from 80° to 150°.

An exemplary method for using the surgical instrument of the present embodiment will be described in the following.

As illustrated in Fig. 1(a), the pair of working sections 3, 3 of the surgical instrument 1 is in closed states. When a practitioner passes his/her fingers through the ring sections 6, 6 and applies a force in a direction of separating the pair of operating sections 4, 4 from each other, the rod-like members 2, 2 pivot around the rotation shaft 5. Then, the working sections 3, 3 separate from each other to open.

In separating living tissues, the surgical instrument 1 is moved from a closed state to an open state, and simultaneously, is inserted between the living tissues to be separated, entering further, thereby separating the living tissues from each other. The separating sections 13, 13, namely, the outer surfaces of the tip sections 8, 8, have rounded shapes, making it possible, at this time, to separate the living tissues from each other without impairing crucial living tissues.

Grasping an object such as a living tissue or a surgical instrument using the grasping surfaces 10, 10 can be performed by disposing the object between the grasping surfaces 10, 10, and then applying a force in the direction of causing the operating sections 4, 4 to come closer to each other. The step sections 12, 12, which include no blades, exist between the grasping surfaces 10, 10 and the cutting edges 11,11. In addition, a virtual plane containing the grasping surfaces 10, 10, and the cutting edges 11, 11, are not on the same plane. Therefore, it is possible to prevent the object from being erroneously disposed and cut between the cutting edges 11, 11.

Cutting an object such as a living tissue or suture or the like is possible by disposing the object between the cutting sections 9, 9, and then, by applying a force in the direction of causing the operating sections 4, 4 to come closer to each other. This causes the cutting edges 11, 11 to slide against each other, making it possible to cut the object using the cutting edges 11,11. In addition, the surgical instrument 1 can be used for completely cutting the object or cutting part of the object with the cutting edges 11, 11.

A conventional surgery has required a dedicated surgical instrument for surgical operations including separating, grasping, and cutting, whereas using a surgical instrument 1 can perform the above-described three surgical operations, making it possible to decrease the types of surgical instruments used in a surgery. As a result, management of surgical instruments can be streamlined, burden on health-care professionals being decreased, making it possible to reduce a total cost.

Also, conventionally, an instrument has been exchanged with a dedicated surgical instrument corresponding to each surgical operation, and a surgical instrument held by a practitioner has been frequently exchanged with another by the practitioner. A surgical instrument 1 can perform the three surgical operations alone, making it possible to decrease the number of times of exchanging surgical instruments by the practitioner, thereby reducing the time taken for a surgery. Reducing the time taken for a surgery not only decreases the burden on a patient by suppressing the amount of bleeding or the like, but also decreases a burden on all health-care professionals involving in the surgery.

A surgical instrument of this invention is not limited to the embodiment described above. Various modifications according to the appended claims are possible as long as they can achieve the object of the present invention.

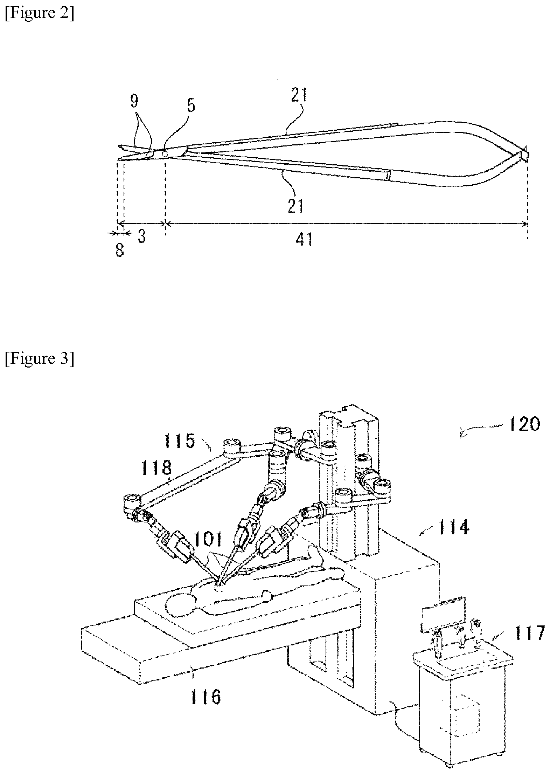

For example, in the surgical instrument 1, fingers are inserted to the ring sections 6, 6 of the pair of operating sections 4, 4 to move the pair of operating sections 4, 4 to be positioned apart from each other and close to each other, thereby opening and closing the working sections 3, 3. Alternatively, as illustrated in Fig. 2, operating sections 41, 41 need not always have the ring sections for inserting the fingers. The operating sections 41, 41 may be configured to be plates curving outwardly and function as springs, whereby a force applied from the outside to the inside closes the working sections 3, 3.

In the above-described embodiment, the surgical instrument 1 has been described when operated by inserting fingers of the practitioner in the ring sections 6, 6 of the operating sections 4, 4. Alternatively, as illustrated in Fig. 3, the surgical instrument of the present disclosure may also be used as a manipulator 101 that is attached to a medical robot system 120.

The configuration of the medical robot system 120 is not particularly limited as long as the system enables surgical operations with the surgical instrument of the present disclosure attached thereto. The medical robot system 120 includes, for example, as illustrated in Fig. 3, a station 114 provided on a floor of a surgery room, a surgical robot 115 provided on the station 114, a movable bed 116 extending from a side of the station 114, and a control apparatus 117 that controls these components. The surgical robot 115 has three arms 118 each having a plurality of joints. To a tip of each arm, the manipulator 101, namely, the surgical instrument of the present disclosure, is detachably attached. The arms 118 and the manipulators 101, being controlled by the control apparatus 117, individually operate using built-in motor drive.

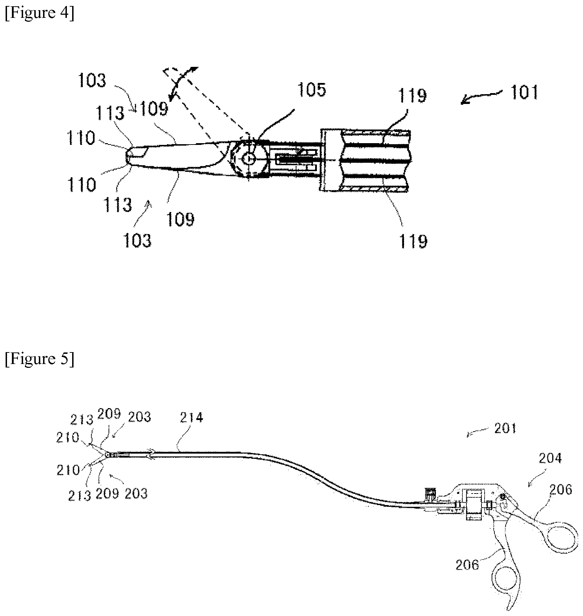

In the manipulator 101, a mechanism for opening and closing a pair of working sections 103 is not particularly limited. As illustrated in Fig. 4, the manipulator 101 is configured, for example, to have the pair of working sections 103, 103 openably/closably coupled to each other on a pulley 105 that is a pivot shaft. To this pulley 105, a drive wire 119 is fixed, the pair of working sections 103, 103 being opened and closed by movements of the drive wire 119 in forward and backward directions. The pair of working sections 103, 103 each has a cutting section 109, a grasping surface 110, and a separating section 113, similarly to the above-described surgical instrument 1.

In a medical robot system using a plurality of types of surgical instruments as manipulators in a surgery, for example, when the number of types of surgical instruments being used is more than the number of existing arms, a manipulator needs to be exchanged with another type of manipulator during the surgery. The surgical instrument of the present disclosure, when used as a manipulator, can perform three surgical operations of separation, grasping, and cutting by one manipulator alone. As a result, it is possible to decrease the number of manipulators being used and decrease the number of times of exchanging the manipulators during the surgery. Accordingly, using the surgical instrument of the present invention as a manipulator can reduce the time taken for a surgery performed by the medical robot system and can suppress operation errors or the like in health-care professionals, thereby decreasing a burden on a patient and health-care professionals.

Furthermore, as illustrated in Fig. 5, the surgical instrument of the present disclosure can be used as an endoscopic surgical instrument 201. As long as the endoscopic surgical instrument 201 includes, at a tip thereof, similarly to the surgical instrument 1 of the above-described embodiment, a pair of working sections 203, 203 including: a cutting section 209; a grasping surface 210; and a separating section 213, the configuration of other than the above is not particularly limited. As illustrated in Fig. 5, for example, the endoscopic surgical instrument 201 includes, in the order from a tip side, the working section 203, a guide pipe section 214, and an operating section 204. To the guide pipe section 214, a drive shaft 215 is inserted, and a proximal end of the guide pipe section 214 is coupled to an opening/closing handle 206 of the operating section 204. The pair of working sections 203, 203 that is coupled to the drive shaft 215 is provided at the tip side of the guide pipe section 214. The pair of working sections 203 is opened and closed by pivoting the opening/closing handle 206.

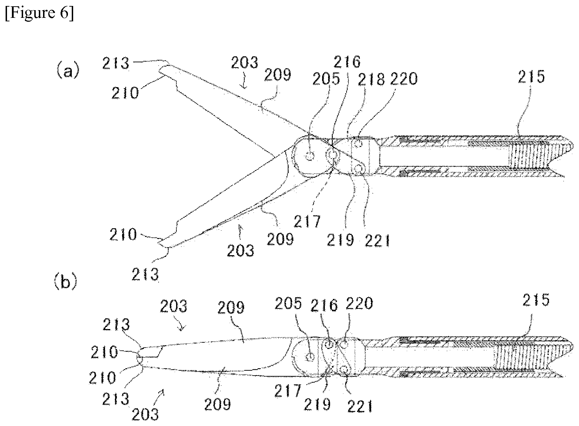

A mechanism for opening and closing the pair of working sections 203 in the endoscopic surgical instrument 201 is not particularly limited. As illustrated in Figs. 6(a) and 6(b), for example, the pair of working sections 203, 203 is openably/closably and pivotally supported by a hinge pin 205 that is the pivot shaft, and by pins 216 and 217. Between the hinge pin 205 and the drive shaft 215, a pair of opening/closing links 218 and 219 is disposed, each link being pivotally supported by the pins 216 and 217 at the tip of the drive shaft 215. The other end of the pair of opening/closing links is pivotally supported by pins 220 and 221 at the proximal end of the pair of working sections 203, 203. Pivoting the opening/closing handle 206 that is coupled to the proximal end of the drive shaft 215 can move the drive shaft 215 in a shaft direction, thereby opening and closing the pair of working sections 203, 203.

An endoscopic surgery is performed by incising an abdominal wall or the like to make a plurality of holes, and a trocar is inserted to the hole. A surgery is performed by inserting a surgical instrument such as an endoscope and forceps through the trocar. Since the number of holes made at incision is minimized to decrease a burden on a patient, a plurality of types of surgical instruments for use is inserted to one hole. The surgical instrument of the present disclosure, when used as an endoscopic surgical instrument, can perform three surgical operations of separation, grasping, and cutting by one endoscopic surgical instrument. As a result, it is possible to decrease the number of surgical instruments being used and decrease the number of times of exchanging the surgical instruments during the surgery. As a result, using the surgical instrument of the present disclosure as an endoscopic surgical instrument can reduce the time needed for an exchange of the surgical instruments, thereby reducing the time taken for a surgery. Furthermore, decreasing complicated exchange operations can suppress errors in health-care professionals, thereby decreasing a burden on a patient and the health-care professionals.

The surgical instrument of the present disclosure is used in a surgery, for example, for separating living tissues from each other, grasping and pulling the living tissue, grasping surgical instruments such as a needle and suture, and cutting the living tissue and suture or the like. The surgical instrument of the present disclosure alone can perform surgical operations including separation, grasping, and cutting. Therefore, the surgical instrument can be used in place of a plurality of dedicated medical devices for individual surgical operations. Therefore, the surgical instrument of the present disclosure is used in a surgery that includes at least one of the surgical operations of separation, grasping, and cutting.

### Reference Signs List

- 1: surgical instrument
- 2: rod-like member
- 3: working section
- 4: operating section
- 5: pivot shaft
- 6: ring section
- 7: handle section
- 8: tip section
- 9: cutting section
- 10: grasping surface
- 11: cutting edge
- 12: step section
- 13: separating section
- 101: manipulator
- 120: medical robot system
- 201: endoscopic surgical instrument

## Claims

1. A surgical instrument (1) having a pair of working sections (3) openably and closably coupled to each other on a pivot shaft (5),
the pair of working sections (3) comprising:
a cutting section (9) configured to cut a first object by sliding a pair of cutting edges (11) against each other;
a pair of grasping surfaces (10) configured to grasp a second object by closing the pair of working sections (3); and
a pair of separating sections (13) configured to be able to separate living tissues when opening the pair of working sections,
wherein the pair of separating sections (13) are outer surfaces of tip sections (8) of the working sections (3) and have elliptical outlines when the pair of working sections (13) are closed and viewed from a tip side of the surgical instrument (1),
wherein a thickness of the pair of separating sections (13) is 1 to 5 mm when the pair of working sections (13) are closed,
wherein the pair of grasping surfaces (10) are configured to be between the pair of separating sections (13) when the pair of working sections (13) are closed, and
wherein the pair of grasping surfaces (10) have a length of 1 to 5 mm in a first direction which is a longitudinal direction of the working section (3) and have a length of 1 to 5 mm in a direction perpendicular to the first direction.

2. The surgical instrument according to claim 1, comprising a step section (12) without a blade between the grasping surface (10) and the cutting edge (11),
wherein a bottom portion of the step section (12) is continuously connected to the grasping surface (10) and a top portion of the step section is continuously connected to the cutting edge (11).

3. The surgical instrument according to claim 1 or 2, wherein, on a pair of rod-like members (2) configured to cross each other on the pivot shaft (5) as an intersection, the pair of working sections (3) is provided on a tip side with respect to the pivot shaft (5), a pair of operating sections (4) is provided on a rear end side with respect to the pivot shaft (5), and the pair of working sections (3) is configured to open and close corresponding to opening and closing of the pair of operating sections (4).

4. The surgical instrument according to any one of claims 1 to 3, wherein a length of each of the cutting edges (11) is from 5 to 50 mm.

## Patentansprüche

1. Chirurgisches Instrument (1) mit zwei Arbeitsabschnitten (3), die an einem Drehzapfen (5) öffnungs- und schließfähig miteinander gekoppelt sind,
wobei die Arbeitsabschnitte (3) aufweisen:
einen Schneidabschnitt (9), der zum Schneiden eines ersten Objekts durch Gleitenlassen von zwei Schneidkanten (11) gegeneinander ausgeführt ist;
zwei Greifflächen (10), die zum Ergreifen eines zweiten Objekts durch Schließen der zwei Arbeitsabschnitte (3) ausgeführt sind; und
zwei Trennabschnitte (13), die ausgeführt sind, um beim Öffnen der zwei Arbeitsabschnitte lebendes Gewebe trennen zu können,
wobei die zwei Trennabschnitte (13) Außenflächen von Spitzenabschnitten (8) der Arbeitsabschnitte (3) sind und elliptische Umrisse haben, wenn die zwei Arbeitsabschnitte (13) geschlossen sind und von einer Spitzenseite des chirurgischen Instruments (1) her betrachtet werden,
wobei eine Dicke der zwei Trennabschnitte (13) 1 bis 5 mm beträgt, wenn die zwei Arbeitsabschnitte (13) geschlossen sind,
wobei die zwei Greifflächen (10) ausgeführt sind, um sich zwischen den zwei Trennabschnitten (13) zu befinden, wenn die zwei Arbeitsabschnitte (13) geschlossen werden, und
wobei die zwei Greifflächen (10) in einer ersten Richtung, die eine Längsrichtung des Arbeitsabschnitts (3) ist, eine Länge von 1 bis 5 mm haben und in einer zur ersten Richtung lotrechten Richtung eine Länge von 1 bis 5 mm haben.

2. Chirurgisches Instrument nach Anspruch 1, das einen Stufenabschnitt (12) ohne eine Klinge zwischen der Greiffläche (10) und der Schneidkante (11) aufweist,
wobei ein unterer Teil des Stufenabschnitts (12) fortlaufend mit der Greiffläche (10) verbunden ist und ein oberer Teil des Stufenabschnitts fortlaufend mit der Schneidkante (11) verbunden ist.

3. Chirurgisches Instrument nach Anspruch 1 oder 2, wobei an zwei stabähnlichen Elementen (2), die ausgeführt sind, um einander am Drehzapfen (5) als Schnittpunkt zu überkreuzen, die zwei Arbeitsabschnitte (3) in Bezug auf den Drehzapfen (5) auf einer Spitzenseite bereitgestellt sind, zwei Handhabungsabschnitte (4) in Bezug auf den Drehzapfen (5) auf einer Seite des hinteren Endes bereitgestellt sind und die zwei Arbeitsabschnitte (3) ausgeführt sind, um sich entsprechend dem Öffnen und Schließen der zwei Handhabungsabschnitte (4) zu öffnen und zu schließen.

4. Chirurgisches Instrument nach einem der Ansprüche 1 bis 3, wobei eine Länge jeder der Schneidkanten (11) von 5 bis 50 mm beträgt.

## Revendications

1. Instrument chirurgical (1) ayant une paire de sections de travail (3) raccordées l'une à l'autre de manière à être capables de s'ouvrir et de se fermer sur un axe de pivotement (5),
la paire de sections de travail (3) comprenant :
une section de coupe (9) configurée pour couper un premier objet en faisant glisser une paire d'arêtes coupantes (11) l'une contre l'autre ;
une paire de surfaces de préhension (10) configurée pour saisir un deuxième objet en refermant la paire de sections de travail (3) ; et
une paire de sections de séparation (13) configurée pour être capable de séparer des tissus vivants quand on ouvre la paire de sections de travail,
dans lequel la paire de sections de séparation (13) sont les surfaces externes des sections formant pointe (8) des sections de travail (3) et ont des contours elliptiques quand la paire de sections de travail (13) sont fermées et visionnées depuis un côté pointe de l'instrument chirurgical (1),
dans lequel une épaisseur de la paire de sections de séparation (13) va de 1 à 5 mm quand la paire de sections de travail (13) sont fermées,
dans lequel la paire de surfaces de préhension (10) sont configurées pour être entre la paire de sections de séparation (13) quand la paire de sections de travail (13) sont fermées, et
dans lequel la paire de surfaces de préhension (10) ont une longueur de 1 à 5 mm dans une première direction qui est une direction longitudinale de la section de travail (3) et ont une longueur de 1 à 5 mm dans une direction perpendiculaire à la première direction.

2. Instrument chirurgical selon la revendication 1, comprenant une section formant cran (12) sans lame entre la surface de préhension (10) et l'arête coupante (11),
dans lequel une partie inférieure de la section formant cran (12) est raccordée de manière continue à la surface de préhension (10) et une partie supérieure de la section formant cran est raccordée de manière continue à l'arête coupante (11).

3. Instrument chirurgical selon la revendication 1 ou la revendication 2, dans lequel, sur une paire d'éléments semblables à une tige (2) configurés pour se croiser sur l'axe de pivotement (5) comme une intersection, la paire de sections de travail (3) est fournie sur un côté pointe par rapport à l'axe de pivotement (5), une paire de sections d'actionnement (4) est fournie sur un côté d'extrémité arrière par rapport à l'axe de pivotement (5), et la paire de sections de travail (3) est configurée pour s'ouvrir et se fermer en correspondance avec l'ouverture et la fermeture de la paire de sections d'actionnement (4).

4. Instrument chirurgical selon l'une quelconque des revendications 1 à 3, dans lequel une longueur de chacune des arêtes coupantes (11) va de 5 à 50 mm.
